Europäisches Patentamt

(19) **European Patent Office**

Office européen des brevets

(11) Publication number: **0 108 580**
**A1**

(12) # EUROPEAN PATENT APPLICATION

(21) Application number: 83306603.8

(22) Date of filing: 28.10.83

(51) Int. Cl.³: **C 12 N 15/00**
**C 12 N 1/12, C 12 N 1/20**
**C 12 N 5/00**

(30) Priority: 08.11.82 US 439736

(43) Date of publication of application:
16.05.84 Bulletin 84/20

(84) Designated Contracting States:
AT BE CH DE FR GB IT LI LU NL SE

(71) Applicant: Standard Oil Company
200 East Randolph Drive
Chicago Illinois 60601(US)

(72) Inventor: Ausich, Rodney Lee
1053 West Ogden Avenue Apt. 346
Naperville Illinois 60540(US)

(74) Representative: Ritter, Stephen David et al,
Mathys & Squire 10 Fleet Street
London EC4Y 1AY(GB)

(54) Method for introducing foreign genes into green algae utilizing T-DNA of agrobacterium.

(57) A genetically engineered green algal cell and a method
for introducing biologically functional foreign genes into
green algae are defined in which the T-DNA sequence of the
*Agrobacterium* plasmid is used as a vector to transfer
prokaryotic or eukaryotic DNA into the algal genome where it
is replicated and expressed.

EP 0 108 580 A1

- 1 -

## METHOD FOR INTRODUCING FOREIGN GENES INTO
## GREEN ALGAE UTILIZING T-DNA OF AGROBACTERIUM

This invention relates to a method for replication and expression of exogenous genes in algae. More particularly, this invention relates to the use of Agrobacterium as a vector in the genetic engineering of green algae.

Algae are a very large group of photosynthetic organisms. The group includes single celled and multi-cellular organisms. There are green, blue-green, red, and brown algae. The organisms have flexible growth requirements and can grow in either marine or fresh water environments. All algae are able to use carbon dioxide and light as their carbon and energy sources. They are among the most efficient organisms to convert solar energy into organic matter. Certain species have measured photosynthetic efficiencies of 1 to 2 per cent in the field, which represents a biomass production of 15-30 g dry wt/m$^2$/day. Goldman, J. G. In: Algae Biomass eds. G. Shelef and C. J. Soeder, 1980, Elsevier/North Holland Biomedical Press, pp. 343-359. Under optimal laboratory conditions, photosynthetic efficiencies of 20 per cent have been reported. Shelef, G.; Oswald, W. J.; and McGauhey, P. H. J. Sanit. Engin. Div. Am. Soc. Civ. Engrs., vol. 96, SA1 (1970), pp. 91-110.

Algae have a varied biosynthetic machinery, and many species produce compounds that have future industrial applications. Some of these chemicals are produced by algae in very high levels. One species synthesizes over 50 per cent of its cell mass as glycerol, and others produce high levels of hydrocarbons, lipids, and protein. In addition, a number of unique compounds are made by algae and have potential applications in many different areas. Aaronson, S.; Berner, T.; and Dubinsky, Z. In: Algae Biomass, supra, pp. 575-601.

It is the biosynthetic capability of algae which makes them a future source for valuable compounds. Characteristics that make algae attractive for industrial purposes include their ability to use simple components such as carbon dioxide and light for growth, and their varied and efficient biosynthetic capability.

The ultimate objective of the genetic engineering process is the transfer of a foreign gene coding for a particular useful characteristic into a new host cell where the foreign gene is then expressed. The transfer of a foreign gene involves several steps. First, the foreign gene coding for the desired characteristic is incorporated into a vector through a gene-splicing procedure. Vectors, or cloning vehicles, are DNA (deoxyribonucleic acid) molecules that physically transport the foreign gene to the new host cell. Vectors are generally either plasmids, which are circular DNA molecules, or viruses, which are linear DNA molecules. The vector-foreign gene hybrid is then transferred to the host cell using a transformation process. The result is a genetically engineered cell with the desired foreign gene now present in the new host cell.

A successfully genetically engineered cell is one in which the foreign gene, and hence the desired characteristic, is "expressed." Gene expression is a multistep process requiring transcription of DNA into mRNA (messenger ribonucleic acid) and translation of mRNA into protein. For these purposes, the DNA is divided into control regions, which mediate expression of the information, and structural regions, which code for proteins. To begin transcription, the host cell RNA-polymerase must "recognize," or bind to, a control region of the DNA (hereinafter referred to as the "promoter"). The promoter sequence is often different depending upon the DNA material used, e.g., prokaryotic DNA v. eukaryotic DNA. The enzyme RNA polymerase moves along the structural gene transcribing the DNA encoded message into mRNA. The mRNA must contain another control sequence, the translation start site, which allows it to bind to the ribosomes where it is translated into protein. Several attempts to create genetic engineering systems have failed because although foreign DNA was successfully transferred to a new host cell, the cell's own transcription and translation systems were unable to recognize the control sequences of the foreign DNA and the foreign gene was not expressed. Hopwood, David A., "The Genetic Programming of Industrial Microorganisms," Scientific American, vol. 245, No. 3, pp. 90-102, September 1981.

There are presently only a few species in which the various steps for genetic engineering have been determined. For example, methods for genetically engineering bacteria are described in U.S. Patent No. 4,259,444 to Chakrabarty, U.S. Patent No. 4,278,765 to Debabov et al., U.S. Patent No. 4,294,927 to Alfoldi et al., U.S. Patent No. 4,237,224 to Cohen et al., U.S. 4,332,892 to Ptashne et al. and U.S. Patent No. 4,338,397 to Gilbert et al. U.S. Patent No. 4,318,987 to Murillo Araujo et al. and Ilgen, Christine; Farabaugh, P. J.; Hinnen, A.; Walsh, Jean M.; and Fink, G. R. In: Genetic Engineering, Vol. I, eds. Jane K. Setlow and Alexander Hollaender (New York, Plenum Press) 1979, pp. 117-132, describe genetic engineering

0108580

-4-

systems for fungi and yeasts.  Methods for genetically engineering in animal cells have also been described. For review see Lloyd H. Graf, Jr., "Gene Transformation," *American* *Scientist*, vol. 70, pp. 496-505, Sept.-Oct. 1982.

Presently, there are no systems for genetic engineering in algae.  There are no vectors, transformation mechanisms, or selection systems available. Accordingly, there is need for a genetic engineering system where foreign DNA is first transferred to, and then expressed in, algae.

*Agrobacterium* are a group of plant pathogens that infect a number of dicotyledonous plants.  These include sunflower, tobacco, and carrot.  The bacteria cannot infect monocotyledonous plants, such as grasses, orchids, or lilies.  During the infection process, a small portion of the bacterial plasmid DNA, the T-DNA, is transferred to the plant cell and is incorporated into the plant genome.  Chilton, Mary-Dell, et al., "Stable Incorporation of Plasmid DNA into Higher Plant Cells:  The Molecular Basis of Crown Gall Tumorigenesis," *Cell*, vol. 11, pp. 263-271, June 1977.  The T-DNA is replicated and expressed causing the plant cell to have a completely altered metabolism.  In addition the infected plant cell synthesizes a variety of anomalous amino acids or opines.

Since DNA is transferred from the bacterial cell to the plant cell, many groups have considered using crown gall bacteria (*Agrobacterium tumefaciens*), a species of *Agrobacterium*, as a vector to genetically engineer plant cells.  In one scheme, a desired gene is attached to the T-DNA region of the Ti plasmid by gene splicing techniques.  The crown gall bacterial strain would be one that can infect and transfer DNA to the plant cell but would not cause the plant cell to become tumorous. The plant cell is then infected with the crown gall bacteria, and the foreign gene is transferred to the plant cell with the T-DNA. The result is a genetically engineered plant cell that contains the foreign gene.

Several groups have been able to transfer foreign genes to plant cells using crown gall bacteria. However, many of these foreign genes have not been expressed by the plant cells. See Garfinkel, David J., et al. "Genetic Analysis of Crown Gall: Fine Structure Map of the T-DNA by Site-Directed Mutagenesis," Cell, vol. 27, pp. 143-153, Nov. 1981. Crown gall bacteria and other species of Agrobacterium, however, have not been used to transfer foreign genes into algal cells. As indicated above, there are presently no systems available for the genetic engineering of algae.

Therefore, it is an object of this invention to create a method for genetically engineering algae in which foreign genes may be transferred into algal cells, replicated, and expressed. It is a further object of this invention to create a genetically engineered algal cell in which foreign DNA is expressed.

The objects of this invention have been attained through a method of introducing foreign genes into green algae utilizing the plasmid DNA of Agrobacterium as a vector. Specifically, foreign genes composed of either eukaryotic or prokaryotic DNA can be transferred into host green algal cells utilizing Agrobacterium T-DNA as a vector wherein the foreign genes are incorporated into the algal genome, and replicated and expressed by the algal biosynthetic machinery. The method can be used to produce a genetically engineered green algal cell in which foreign DNA is incorporated, replicated and expressed.

I have found that Agrobacterium is able to infect green algae in a process similar to the process in which it infects higher plants. This is surprising in view of the previously recognized host range of Agrobacterium which excluded all lower plants. See, for example, De Cleene, Marcel and De Lev, Jozef, "The Host Range of Crown Gall" The Botanical Review, vol. 42(4), 1976, pp. 389-466. It is also surprising that green algae are infected considering Agrobacterium is apparently unable to infect blue-green algae as is evidenced in the Examples below. One would not expect from the limited documented host range of Agrobacterium and its inability to infect blue-green algae, that the bacteria would infect green algae. However, I have found that Agrobacterium is able to infect green algae and transfer bacterial T-DNA into the algal genome.

The first step of the invention requires the incorporation of foreign DNA into the vector. Because it is the T-DNA section of the Agrobacterium plasmid DNA which is incorporated into the genome of the infected cell, it is essential that the gene or genes to be transferred are incorporated within the T-DNA sequence. The desired foreign gene or genes can be inserted into the T-DNA using gene splicing procedures known in the art. One such procedure is described in Garfinkel, David J., et al., "Genetic Analysis of Crown Gall: Five Structure Map of the T-DNA by Site-Directed Mutagenesis," Cell, vol. 27, pp. 143-153, November, 1981, which is incorporated by reference.

The foreign genes which are inserted into the DNA can be composed of either eukaryotic or prokaryotic DNA. The biosynthetic machinery of green algae is able to recognize promoter sequences and express genes from both systems unlike green plants which recognize only eukaryotic promoters.

A wide variety of genes can be employed as the foreign genes from a wide variety of sources. Any intact gene can be employed which can be bonded to the plasmid vehicle.

One group of proteins which are directly useful are hormones. Illustrative hormones include parathyroid hormone, growth hormone, gonadotrophins, (FSH, luteinizing hormone, chorionogonadotrophin, and glycoproteins), insulin, ACTH, somatostatin, prolactin, placental lactogen, melanocyte stimulating hormone, thyrotropin, parathyroid hormone, calcitonin, enkephalin, and angiotensin.

Other proteins of interest include serum proteins, fibrinogin, prothrombin, thromboplastin, globulin, e.g., gamma-globulins or antibodies, heparin, antihemo-philia protein, oxytocin, albumins, actin, myosin, hemoglobin, ferritin, cytochrome, myoglobin, lactoglobulin, histones, avidin, thyroglobulin, interferin, kinins and transcortin.

Where the gene or genes produce one or more enzymes, the enzymes may be used for fulfilling a wide variety of functions. Included in these functions are nitrogen fixation, production of amino acids, e.g., polyiodothyronine, particularly thyroxine, vitamins, both water and fat soluble vitamins, antimicrobial drugs, chemotherapeutic agents, e.g., antitumor drugs, polypeptides and proteins e.g. enzymes from apoenzymes and hormones from prohormones, diagnostic reagents, energy producing combinations, e.g., photosynthesis and hydrogen production, prostaglandins, steroids, cardiac glycosides, coenzymes, and the like.

The enzymes can be individually useful as agents, separate from the algal cell for commercial applications, e.g., in detergents, synthetic transformations, diagnostic agents and the like. Enzymes are classified by the I.U.B. under the classifications as I. Oxidoreductases; II. Transferases; III. Hydrolases; IV. Lyases; V. Isomerases; and VI. Ligases.

It is preferable when inserting foreign genes into the vector that a selection marker also be inserted. A selection marker is a gene or set of genes which codes for a characteristic which allows for separation of cells which have been successfully genetically engineered from those which have not. Selection markers are well-known in the art and include sequences which confer antibiotic resistance, sequences which detoxify and sequences restoring prototrophy. The selection system to be used in a particular system will be dependent upon several factors including, but not limited to, the algal strain used and the desired end product.

Once the vector has been prepared, algae are infected by the _Agrobacterium_ strain containing the engineered plasmid. It has been found in plant cells that metabolically active bacterial cells produce more tumors. Therefore, it is preferable to use _Agrobacterium_ cultures which are in the mid- to late-log phase. Cells in the cultures should be washed with sterile algal growth medium to remove bacterial growth medium in order to prevent bacteria from overgrowing the algae when algal cells are plated on selective medium. In plants, the cells must be actively dividing in order to be infected by the bacteria. It is therefore preferable to use algal cells which are in the actively dividing mode or mid- to late-log phase. Cultures of the bacteria and algae are mixed together on algal growth medium. Cells are allowed to incubate for 30 seconds to 15 minutes, preferably 5 minutes, at less than 32°C.

Agrobacterium are not known to infect plants at temperatures of 32°C or higher. The actual temperature for infection is preferably the optimum temperature for growth of the particular algal species employed. Significantly lower temperatures would not allow the algal cells to actively divide, a necessary condition for infection. During infection the T-DNA of the bacterial plasmid is incorporated into the algal DNA where it is replicated and expressed.

Agrobacterium can be used as a vector in the engineering of all green algae. Green alga include the members of the chlorophyta phylum. This phylum includes, for example, the genera Chlamydomonas, Dunaliella, and Chlorella. This phylum particularly includes Chlamydomonas reinhardtii, Protosiphon botryoides, and Chlorella pyrenoidosa.

Currently, several species of Agrobacterium have been identified which can be used in this process. Species which would be preferable due to extensive information which has been compiled regarding the genetic compositions of their plasmids and their infection processes include Agrobacterium rhizogenes and Agrobacterium tumefaciens.

EXAMPLES

In examples listed below, the following cultures and conditions were used.

The green alga Chlamydomonas reinhardtii (UTEX 2246) was grown on CA medium (Table 1).

The green alga Protosiphon botryoides (Cambridge Culture Collection 731/1a) was grown on 625 medium (Table 1).

The blue-green alga Anacystis nidulans (ATCC 27344) was grown on 625 medium (Table 1).

The 320 strain of Agrobacterium tumefaciens was obtained from Drs. Eugene Nester and Milton Gordon (Garfinkel et al., 1981) and was grown on nutrient medium.

The aforementioned strains are all available to the public  strains UTEX 2246, CCC 731/1A and ATCC 27344 having been made available through the respective depositories.  The 320 strain may be obtained from Drs Eugene Nester and Milton Gordon, Department of Biochemistry, University of Washington, Seattle, Washington 98195.

The  320 strain contains the transposon Tn5 in the T-DNA region of the Ti plasmid.  Tn5 is a prokaryotic gene that codes for the protein aminoglycoside phosphotransferase (3')-II (APH).  This protein detoxifies the antibiotics neomycin, kanamycin, and G418 by transferring the gamma photosphate group of ATP to the antibiotic.  These antibiotics are toxic to many different species.  Some bacteria and yeast cells which contain the Tn5 gene are able to grow in the presence of the antibiotics.

The aqueous extract from agar was prepared by stirring 10 g of Difco-Bacto agar in 1 litre of water for 2 hours.  The suspension was filtered through 2 layers of Miracloth.  One liter of CA agar medium was made using 1 liter aqueous extract.

-11-

Table 1

Algae Growth Media

CA Medium

| | mg/l |
|---|---|
| $NH_4Cl$ | 50 |
| $MgSO_4\ 7H_2O$ | 20 |
| $CaCl_2\ 2H_2O$ | 10 |
| $K_2HPO_4$ | 720 |
| $KH_2PO_4$ | 360 |
| NaAcetate | 2000 |

Plus 1 ml of Hutners trace elements per liter medium:

| | g/l |
|---|---|
| $Na_2EDTA$ | 50 |
| $H_3BO_3$ | 11.4 |
| $ZnSO_4\ 7H_2O$ | 22 |
| $MnCl_2\ 4H_2O$ | 5.1 |
| $FeSO_4\ 7H_2O$ | 5 |
| $CoCl_2\ 6H_2O$ | 1.6 |
| $CuSO_4\ 5H_2O$ | 1.6 |
| $(NH_4)_6Mo_7O_{24}\ 4H_2O$ | 1.1 |

625 Medium

| | mg/l |
|---|---|
| $NaNO_3$ | 496 |
| $K_2HPO_4$ | 39 |
| $MgSO_4\ 7H_2O$ | 75 |
| $CaCl_2\ 2H_2O$ | 36 |
| Fe citrate | 6 |
| $Na_2SiO_3\ 9H_2O$ | 58 |
| $Na_2CO_3$ | 20 |
| Citric acid | 6 |
| $Na_2EDTA$ | 1 |

Ph 7.5

-12-

EXAMPLE I

A 50 ml. culture of the 320 strain was grown to mid- to late-log phase. The cells were collected by centrifugation and washed twice with sterile CA medium. The cells were resuspended in 5 ml. of medium. About $10^6$ cells from 2-day-old Chlamydomonas cultures (mid- to late-log phase) were mixed with 0.2 ml. of the bacterial suspension at room temperature. The mixture was plated onto CA medium with 10 mg/l G418 sulfate (potency 500 mcg/mg), 25 mg/l streptomycin, and 25 mg/l kanamycin.

The plates were incubated at 27°C. 27°C was chosen because it is the optimum temperature for growth of Chlamydomonas. Untreated cells from the alga Chlamydomonas reinhardtii were killed when grown in the presence of the antibiotic G418. Algal colonies composed of the algal cells mixed with the Agrobacterium strain became visible within 4-5 days and grew vigorously in the presence of the antibiotic. The results indicate that the 320 strain infected and transferred the Tn5 gene to the algae and the algae were able to express the Tn5 gene to make a functional protein. Colonies of the Agrobacterium infected Chlamydomonas were isolated and grown on CA agar medium containing 10 mg/l G418 and 10 mg/l carbenicillin.

The infected Chlamydomonas cells also showed other indications of altered metabolism. Whereas normal Chlamydomonas cells grow equally well on liquid or solid media, infected cells would grow only in a liquid medium that had been supplemented with an aqueous extract of the Difco-Bacto agar. Cells, which normally grow as single cells, grew as a clumped mass. Alterations in morphology were attributed either to the expressions of other regions of the T-DNA or the disruption of a normal algal gene or genes due to insertion of the T-DNA.

To further substantiate this evidence, several tests were performed on the infected cells. Proteins were isolated and assayed from cultures of infected Chlamydomonas cells from which crown gall bacterial cells had been removed. Results show the presence of the functional APH protein which confers antibiotic resistance indicating that the Tn5 gene had been transferred and expressed. Protein assays also indicated the presence in the algal cells of octopine synthase. Octopine synthase is protein which is coded for by the T-DNA and which contains a eukaryotic promoter.

Analysis of the APH and the octopine synthase proteins by SDS polyacrylamide gel electrophoresis revealed that these proteins were properly synthesized by the algal biosynthetic system. The infected Chlamydomonas cells were able to synthesize faithfully two genes of bacterial origin: the Tn5 gene and the octopine synthase gene. The Tn5 gene contains a bacterial promoter, and the octopine synthase gene presumably contains a plant promoter, since the gene is expressed only in Agrobacterium infected plant cells. The Chlamydomonas cells were able to recognize both types of promoters. This characteristic makes Chlamydomonas a very desirable host for gene transfer experiments.

DNA hybridization assays were also performed to ascertain incorporations of the Agrobacterium DNA into the algal DNA. Results indicate the presence of T-DNA in the infected Chlamydomonas cell DNA.

## EXAMPLE II

As in Example I, a 50 ml. culture of the 320 strain was grown to mid to late log phase. The

-14-

cells were collected by centrifugation and washed twice with sterile 625 media. The cells were resuspended in 5 ml. of media.

For the infection of Protosiphon, about $10^6$ cells from 4-6-day-old cultures (mid- to late-log phase) were mixed with 0.2 ml. of the bacterial suspension. The mixture was incubated at room temperature for five minutes and was then plated onto 625 medium supplemented with 100 mg/l kanamycin and 25 mg/l streptomycin and allowed to grow at 27°C. Colonies became visible in about 7-10 days.

Kanamycin is toxic to the Protosiphon cells, and the untreated algal cells did not grow on the selective medium. The infected algal cells grew in the presence of the antibiotic. These results indicate that the 320 crown gall bacterial strain infected and transferred the Tn5 gene to Protosiphon and that the algal cells were able to express the Tn5 gene to make a functional protein.

## EXAMPLE III

As in Example I, a 50 ml. culture of the 320 strain was grown to mid- to late-log phase. The cells were collected by centrifugation and washed twice with sterile 625 media. The cells were resuspended in 5 ml. of media.

For the infection of Anacystis, about $10^6$ cells from 2-3-day-old cultures (mid- to late-log phase) were mixed with 0.2 ml. of the bacterial suspension. The mixture was incubated at room temperature for five minutes and was then plated onto 625 media supplemented with 10 mg/l kanamycin and allowed to grow at 27°C. No algal colonies were formed after four weeks.

These results indicate that Agrobacterium was unable either to infect blue-green algae or transfer a functional gene into the algal cells.

The above examples are presented for the purpose of illustration only and are not intended to be limitations of the invention. Additional uses of the methods and products will be apparent to those skilled in the art.

Thus for example strains of green algae and Agrobacterium sp. other than those specifically referred to in the examples may be employed.

CLAIMS:

1.    A method for introducing foreign DNA into green algae comprising: infecting a green algal cell with genetically altered Agrobacterium which contains a biologically functional foreign gene incorporated within the T-DNA sequence of the bacterial plasmid.

2.    The method of Claim 1 wherein the Agrobacterium is selected from Agrobacterium tumefaciens and Agrobacterium rhizogenes.

3.    The method of Claim 1 or Claim 2 wherein the foreign DNA is selected from eukaryotic DNA, prokaryotic DNA and combinations thereof.

4.    The method of any preceding claim wherein the green algae comprises algae chosen from the species Chlamydomonas reinhardtii, Protosiphon botryoides, and Chlorella pyrenoidosa.

5.    The method of any preceding claim wherein the foreign DNA comprises a selection marker.

6.    A genetically engineered green algal cell containing the T-DNA sequence of an Agrobacterium plasmid wherein said T-DNA contains a biologically functional foreign gene.

7.    A genetically engineered green algal cell according to Claim 6 wherein the Agrobacterium is selected from the group consisting of Agrobacterium tumefaciens and Agrobacterium rhizogenes.

8.    A genetically engineered green algal cell according to Claim 6 or Claim 7 wherein the foreign DNA is selected from the group consisting of eukaryotic DNA, prokaryotic DNA and combinations thereof.

9.    The composition of matter of Claim 11 wherein the green algae comprises algae chosen from the species Chlamydomonas reinhardtii, Protosiphon botoryoides, and Chlorella pyrenoidosa.

10.    A genetically engineered green algal cell according to any of Claims 7 to 9 wherein the foreign DNA comprises a selection marker.

# EUROPEAN SEARCH REPORT

Application number

## DOCUMENTS CONSIDERED TO BE RELEVANT

EP 83306603.8

| Category | Citation of document with indication, where appropriate, of relevant passages | Relevant to claim | CLASSIFICATION OF THE APPLICATION (Int Cl. ³) |
|---|---|---|---|
| A | FR - A - 2 438 087 (SNAM PROGETTI S.P.A.)<br><br>* Page 3, line 21 - page 4, line 36 *<br><br>-- | 1 | C 12 N 15/00<br>C 12 N 1/12<br>C 12 N 1/20<br>C 12 N 5/00 |
| A | DE - B - 1 211 017 (KABUSHIKI KAISHA YAKULT HONSHA)<br><br>* Claim *<br><br>-- | 1,4,9 | |
| A,D | US - A - 4 259 444 (CHAKRABARTY)<br><br>* Abstract *<br><br>-- | 1 | |
| A,D | US - A - 4 237 224 (COHEN et al.)<br><br>* Abstract *<br><br>---- | 1 | |

TECHNICAL FIELDS SEARCHED (Int Cl. ³)

C 12 N

The present search report has been drawn up for all claims

| Place of search | Date of completion of the search | Examiner |
|---|---|---|
| VIENNA | 02-02-1984 | WOLF |